# EUROPEAN PATENT APPLICATION

(11) **EP 4 257 061 A1**
(43) Date of publication of application: **11.10.2023**
(21) Application number: 21903377.6
(22) Date of filing: 06.12.2021
(51) Int. Cl.: A61B 17/02

(54) **TREATMENT PART TRACTION MEMBER FOR ENDOSCOPE**

(30) Priority: 07.12.2020 JP 2020202679
(71) Applicant: Jokoh Co., Ltd, Tokyo 113-0033 (JP); St. Marianna University School of Medicine, Kawasaki-shi Kanagawa 216-8511 (JP); Seiko Paching Co., Ltd., Tokyo 125-0054 (JP)
(72) Inventor: MATSUO, Yasumasa, Kawasaki-shi Kanagawa 216-8511 (JP); HIRAI, Shuumei, Tokyo 125-0054 (JP); MINAI, Hironobu, Kawasaki-shi, Kanagawa 213-8588 (JP)
(74) Representative: AWA Sweden AB
(86) International application number: PCT/JP2021/044793
(87) International publication number: WO 2022/124277

(57) **Abstract**

An endoscopic treatment portion traction member where in improving the traction member that lifts the tissue to be resected from the surrounding tissue to assist in the resection of the tissue to be resected during endoscopic treatment, the ease of grasping the traction member with endoscopic forceps and the ease of handling it in the area to be treated are improved. The endoscopic treatment portion traction member is gripped by the forceps inserted into the endoscope to be inserted into the body, and is fixed to the tissue to be resected to lift the tissue from the surrounding tissue. The endoscopic traction member includes an annular body; and an outer tongue provided at one or two locations in the peripheral portion of the annular body to be gripped by forceps, and is formed from elastic resin.

## Description

### 1. Field of the Invention

The present invention relates to an endoscopic treatment portion traction member. More specifically, the present invention relates to a traction member for lifting the tissue to be resected from the surrounding tissue to assist in the resection of the tissue to be resected during endoscopic treatment.

### 2. Description of Related Art

Improvements in endoscope performance have allowed endoscopes to be used not only for examination of internal tissues but also for tissue sampling and even tissue resection recently. As an example of endoscopic surgery, endoscopic submucosal dissection (ESD) allows a tumor tissue (lesion site) of early-stage cancer that has developed on the mucosa within the digestive tract such as the colon to be minimally invasively resected.

In the ESD treatment, the lesion site to be resected is first identified. Next, saline or other solution is injected into the submucosal layer immediately below the lesion site, and the lesion to be resected is lifted from the surrounding tissue. Then, incision of the mucosa around the lesion site and dissection of the submucosal layer are performed. When the submucosal layer around the lesion site is incised and resected, the lifted lesion site is not sufficient to accurately incise the area around the lesion site in the limited field of view of the endoscope. Accordingly, the lesion site (and its surroundings) needs to be further lifted in the intestinal tract.

In lifting the lesion site, a traction member including clips that grip the tissue surface and the lesion site in the intestinal tract has been proposed (see, e.g., patent documents 1 and 2). However, the traction members proposed in patent documents 1 and 2 are complex in structure, and the overall size causes the traction member to be hard to be manipulated at the treatment site using forceps incorporated into an endoscope.

Accordingly, a simplified traction member with the clips separate from the traction member has been proposed (see patent document 3). The traction member in patent document 3 is configured to have a plurality of interconnected rings, achieving a certain effect in hanging with the clips. Since the traction member in patent document 3 is configured to have a plurality of interconnected rings, however, the strength of the interconnected portions of the rings cannot be sufficiently maintained. Another problem is in the positions of the member at a time when the rings are bundled together and gripped by the forceps.

### [Prior Art Documents]

### [Patent Documents]

[Patent Document 1] Japanese Laid-Open Patent Publication No. 2008-62004
[Patent Document 2] Japanese Laid-Open Patent Publication No. 2005-103107
[Patent Document 3] Japanese Laid-Open Patent Publication No. 2019-118718

### SUMMARY OF THE INVENTION

As a result of diligent study, the applicant has completed an endoscopic treatment portion traction member that satisfies ease of gripping the traction member by endoscopic forceps and ease of handling in the area to be treated.

The present invention has been made in view of the above-mentioned points, and provides an endoscopic treatment portion traction member where in improving the traction member that lifts the tissue to be resected from the surrounding tissue to assist in the resection of the tissue to be resected during endoscopic treatment, the ease of grasping the traction member with endoscopic forceps and the ease of handling it in the area to be treated are improved.

### [Means to Solve the Problems]

That is, in an embodiment, an endoscopic treatment portion traction member is gripped by forceps inserted into an endoscope to be inserted into the body, and is fixed to the tissue to be resected to pull the tissue to be resected from the surrounding tissue. The endoscopic treatment portion traction member is characterized by including an annular body; an outer tongue provided in a peripheral portion of the annular body and gripped by the forceps.

Further, the outer tongue may be provided at one or two locations in the peripheral portion of the annular body.

Moreover, the outer tongue may include a tapered portion.

Furthermore, the tip end of the outer tongue may be chamfered in an arcuate shape.

In addition, the annular body may be circular in shape and the maximum length of the outer tongue may be 1/10 to 6/10 of the diameter of the peripheral portion of the circular annular body.

Further, the annular body may be oval in shape, and the maximum length of the outer tongue may be 1/10 to 3/10 of the minor axis length of the peripheral portion of the oval-shaped annular body.

Moreover, the annular body may be circular in shape and the diameter of the peripheral portion of the annular body may be 5 to 30 mm.

Furthermore, an inner tongue section may be provided in the inner circumferential portion of the annular body opposite the outer tongue.

In addition, the endoscopic treatment portion traction member may be formed from an elastic resin.

Further, the outer tongue may include a tongue pore.

Moreover, when the endoscopic treatment portion traction member is fixed to the tissue to be resected, the endoscopic treatment portion traction member may be suspended via a clip secured to the tissue to be resected.

### [Advantageous Effects of the Invention]

According to the endoscopic treatment portion traction member of the present invention, the endoscopic treatment portion traction member, which is gripped by forceps inserted into the endoscope and inserted into the body, is fixed to the tissue to be resected to lift the tissue from the surrounding tissue. The member includes an annular body; and an outer tongue provided around the peripheral portion of the annular body and gripped by the forceps. This improves, during the treatment using the endoscope, the ease of gripping the traction member by the endoscopic forceps and the ease of handling in the area to be treated.

### BRIEF DESCRIPTION OF THE DRAWINGS

Features, advantages, and technical and industrial significance of exemplary embodiments of the invention will be described below with reference to the accompanying drawings, in which like numerals denote like elements, and wherein:
FIG. 1 is an overall plan view illustrating an endoscopic treatment portion traction member according to a first embodiment,
FIG. 2(A) is a diagram illustrating the endoscopic treatment portion traction member before it is gripped by forceps, and
FIG. 2(B) is a diagram illustrating the endoscopic treatment portion traction member after it is gripped,
FIG. 3(A) is a diagram illustrating the endoscopic treatment portion traction member when it is inserted into an inside of an endoscope, and
FIG. 3(B) is a diagram illustrating the endoscopic treatment portion traction member when it is exposed from the endoscope,
FIG. 4(A) is a vertical cross-sectional view illustrating the endoscopic treatment portion traction member when it is advanced into the inside of the endoscope, and
FIG. 4(B) is a horizontal cross-sectional view illustrating the endoscopic treatment portion traction member when it is advanced into the inside of the endoscope,
FIG. 5(A) is a first schematic diagram illustrating an endoscopic submucosal dissection, and
FIG. 5(B) is a second schematic diagram illustrating the endoscopic submucosal dissection,
FIG. 6 is a third schematic diagram illustrating the endoscopic submucosal dissection,
FIG. 7(A) is an overall plan view illustrating an endoscopic treatment portion traction member according to a second embodiment,
FIG. 7(B) is an overall plan view illustrating an endoscopic treatment portion traction member according to a third embodiment, and
FIG. 7(C) is an overall plan view illustrating an endoscopic treatment portion traction member according to a fourth embodiment,
FIG. 8(A) is an overall plan view illustrating an endoscopic treatment portion traction member according to a fifth embodiment,
FIG. 8(B) is an overall plan view illustrating an endoscopic treatment portion traction member according to a sixth embodiment,
FIG. 8(C) is an overall plan view illustrating an endoscopic treatment portion traction member according to a seventh embodiment, and
FIG. 8(D) is an overall plan view illustrating an endoscopic treatment portion traction member according to an eighth embodiment,
FIG. 9(A) is an overall plan view illustrating an endoscopic treatment portion traction member according to a ninth embodiment,
FIG. 9(B) is an overall plan view illustrating an endoscopic treatment portion traction member according to a tenth embodiment, and
FIG. 9(C) is an overall plan view illustrating an endoscopic treatment portion traction member according to an eleventh embodiment,
FIG. 10(A) is an overall plan view illustrating an endoscopic treatment portion traction member according to a twelfth embodiment,
FIG. 10(B) is an overall plan view illustrating an endoscopic treatment portion traction member according to a thirteenth embodiment, and
FIG. 10(C) is an overall plan view illustrating an endoscopic treatment portion traction member according to a fourteenth embodiment,
FIG. 11(A) is an overall plan view illustrating an endoscopic treatment portion traction member according to a fifteenth embodiment, and
FIG. 11(B) is an overall plan view illustrating an endoscopic treatment portion traction member according to a sixteenth embodiment,
FIG. 12(A) is an overall plan view illustrating an endoscopic treatment portion traction member according to a seventeenth embodiment, and
FIG. 12(B) is a perspective view illustrating the endoscopic treatment portion traction member when forceps are inserted through the endoscopic treatment portion traction member,
FIG. 13(A) is an overall plan view illustrating an endoscopic treatment portion traction member according to an eighteenth embodiment,
FIG. 13(B) is an overall plan view illustrating an endoscopic treatment portion traction member according to a nineteenth embodiment, and
FIG. 13(C) is an overall plan view illustrating an endoscopic treatment portion traction member according to a twentieth embodiment,
FIG. 14(A) is an overall plan view illustrating an endoscopic treatment portion traction member according to a twenty-first embodiment,
FIG. 14(B) is an overall plan view illustrating an endoscopic treatment portion traction member according to a twenty-second embodiment,
FIG. 14(C) is an overall plan view illustrating an endoscopic treatment portion traction member according to a twenty-third embodiment, and
FIG. 14(D) is an overall plan view illustrating an endoscopic treatment portion traction member according to a twenty-fourth embodiment,
FIG. 15(A) is an overall plan view illustrating an endoscopic treatment portion traction member according to a twenty-fifth embodiment,
FIG. 15(B) is an overall plan view illustrating an endoscopic treatment portion traction member according to a twenty-sixth embodiment, and
FIG. 15(C) is an overall plan view illustrating an endoscopic treatment portion traction member according to a twenty-seventh embodiment,
FIG. 16(A) is an overall plan view illustrating an endoscopic treatment portion traction member according to a twenty-eighth embodiment,
FIG. 16(B) is an overall plan view illustrating an endoscopic treatment portion traction member according to a twenty-ninth embodiment, and
FIG. 16(C) is an overall plan view illustrating an endoscopic treatment portion traction member according to a thirtieth embodiment,
FIG. 17(A) is an overall plan view illustrating an endoscopic treatment portion traction member according to a thirty-first embodiment, and
FIG. 17(B) is an overall plan view illustrating an endoscopic treatment portion traction member according to a thirty-second embodiment.

### DETAILED DESCRIPTION OF EMBODIMENTS

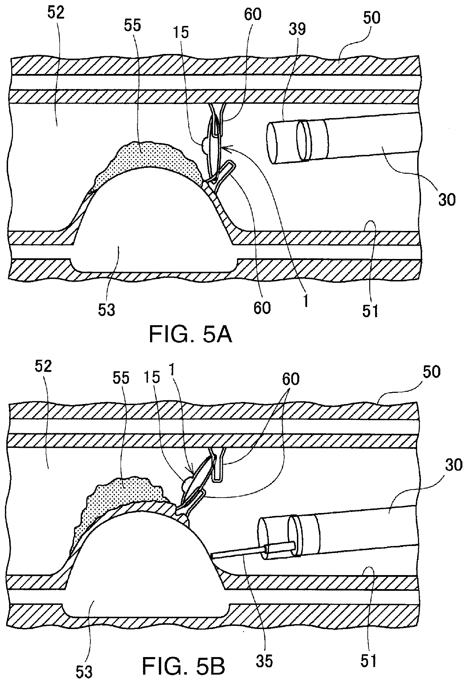

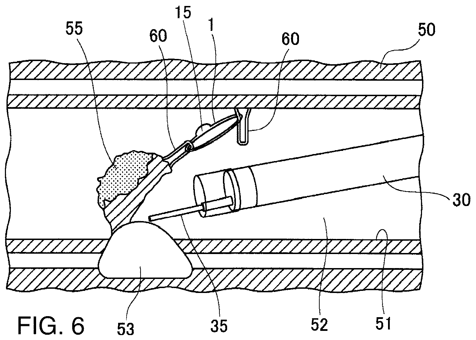

The endoscopic treatment portion traction member according to an embodiment of the present invention is directed to a member exclusively used during endoscopic submucosal dissection, which member is fixed to a tissue to be resected, and lifts and pulls the tissue from the surrounding tissue. Endoscopic submucosal dissection mainly targets the resection of tumor tissue (lesion site) of early-stage cancerous growths on the mucosa inside the digestive tract including the colon (such as rectum, S-colon, descending colon, transverse colon, ascending colon, cecum). Further, the endoscopic treatment portion traction member can be applied to any portion or organ into which an endoscope can be inserted. Examples may include the nasal cavity, cervix uteri, and uterine head. The endoscopic submucosal dissection will be described with reference to FIGS. 5 and 6 below.

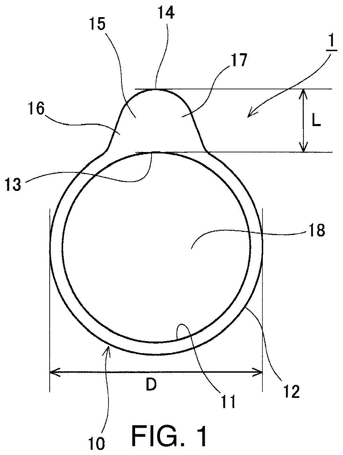

FIG. 1 is an overall plan view of an endoscopic treatment portion traction member 1 according to a first embodiment. The endoscopic treatment portion traction member 1 includes a circular annular body 10 and an outer tongue 15 protruding outward from the annular body 10. The annular body 10 is configured by an inner circumferential portion 11 and a peripheral portion 12, and an inner circular portion 18 is formed inside the annular body 10. The diameter (D) of the peripheral portion 12 of the circular annular body 10 is 5 to 30 mm, preferably 10 to 30 mm. As described below, the endoscopic treatment portion traction member 1 is inserted into the endoscope for treatment on the lesion site. The diameter is specified in consideration of the size at that time. The annular body 10 of the endoscopic treatment portion traction member 1 according to the embodiment is circular. In addition to this, the annular body 10 can be oval-shaped.

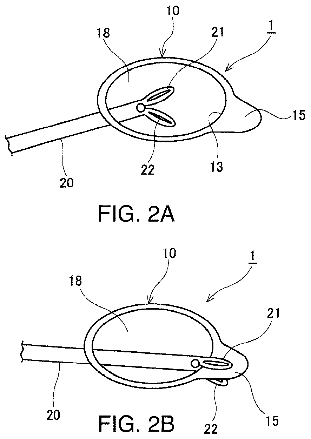

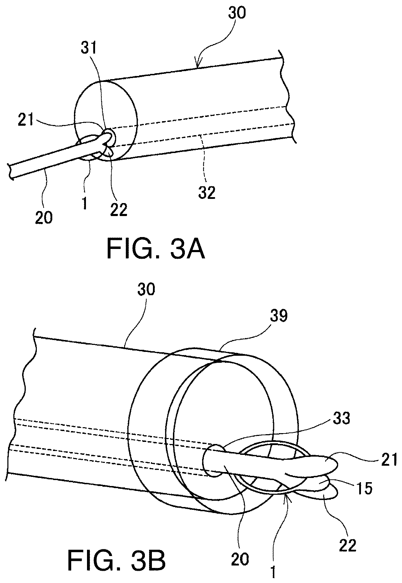

In the endoscopic treatment portion traction member 1 according to the first embodiment, the tongue 15 is provided at one location in the peripheral portion 12 of the annular body 10. The outer tongue 15 is gripped by forceps 20 (see FIGS. 2 and 3, for example) that are inserted into the endoscope 30. In consideration of convenience of grasping, it may be desirable for the outer tongue to be present at various locations around the peripheral portion of the annular body. However, the endoscopic treatment portion traction member needs to pass through a narrow conduit of the endoscope. The extra outer tongue then becomes a resistance during the passage of the member through the conduit, hindering the smooth passage thereof. Accordingly, as in the endoscopic treatment portion traction member 1 according to the embodiment, the outer tongue 15 is provided at one installation position 13 in the peripheral portion 12 of the annular body 10. In the second and other embodiments described below, the outer tongue is provided at two positions opposite to each other (180° orientation) of the peripheral portion 12. Even if the tongues are provided in the opposite positions, the resistance during the passage of the member through the conduit is reduced.

The maximum length (L) of the outer tongue 15 is specified in the range of 1/10 to 6/10, preferably 1/10 to 3/10 of the diameter of the peripheral portion 12 of the annular body 10. The length of the outer tongue 15 is acceptable if it is large enough to be gripped by the endoscopic forceps. If the outer tongue 15 is too small, the endoscopic forceps cannot easily grasp it. If it is significantly large, it may cause resistance to the passage in the endoscope through the conduit, which is undesirable.

As understood from the plan view in FIG. 1, the tip end 14 of the outer tongue 15 is chamfered to be arcuate. In addition, tapered portions 16, 17 are provided on the left and right sides of the outer tongue 15. The tapered portions 16, 17 taper from the installation position 13 side of the annular body 10 towards the tip end 14. The shapes are employed as features to reduce the resistance of the endoscopic treatment portion traction member 1 gripped by the forceps as it passes through the conduit of the endoscope.

FIG. 2(A) illustrates the endoscopic treatment portion traction member 1 when it is gripped by the forceps 20 to be inserted into the endoscope. The forceps 20 are passed through the inside of the inner circular portion 18 for the installation position 13 of the annular body 10. Forceps jaws 21, 22 are provided at the tip ends of the forceps 20, and the opening and closing operations of the forceps jaws 21, 22 allow the object to be gripped. The forceps jaws 21, 22 in FIG. 2(A) are in the open state.

FIG. 2(B) illustrates the endoscopic treatment portion traction member 1 being gripped by the forceps 20. The forceps jaws 21, 22 of the forceps 20 are closed, and the outer tongue 15 is pinched by the forceps jaws 21, 22. The outer tongue 15 is a spreading plate portion of the annular body 10, facilitating the forceps jaws 21, 22 of the forceps 20 to grip the portion. In addition, the entire outer tongue 15 is easily retained in the grasped state. In this state, the endoscopic treatment portion traction member 1 is gripped by the forceps 20 and inserted into the endoscope.

FIG. 3(A) illustrates the endoscopic treatment portion traction member 1 being inserted into the insertion port 31 of the endoscope 30 with the endoscopic treatment portion traction member 1 being held by the forceps 20 in FIG. 2(B) above. The endoscope 30 shown in the FIG. 3(A) has functions for endoscopic submucosal dissection and other procedures. Accordingly, the endoscope 30 includes a conduit 32 for inserting and removing the forceps for resection, suturing, and other procedures in addition to photographing the operating field.

FIG. 3(B) illustrates the endoscopic treatment portion traction member 1 exposed through the opening 33 of the endoscope with the endoscopic treatment portion traction member 1 still held by the forceps 20. As disclosed in FIG. 1, since the endoscopic treatment portion traction member 1 is shaped to be subject to less resistance, it can be gripped by the forceps jaws 21, 22 and advanced through the conduit 32 without resistance. FIG. 3B shows a tip hood 39 of the endoscope 30.

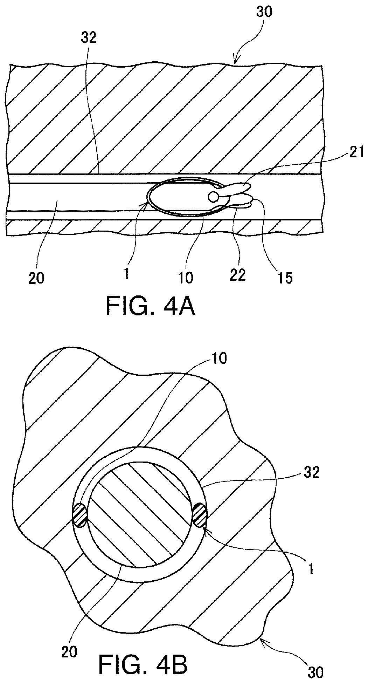

FIG. 4(A) is a longitudinal sectional view of the endoscope in the longitudinal direction illustrating the endoscopic treatment portion traction member 1 being gripped by the forceps jaws 21, 22 of the forceps 20 and advanced in the conduit 32. The diameter of the body of the forceps 20 is less than the inner diameter of the conduit 32. In the case of the endoscope shown in FIG. 4(A), the inner diameter of the conduit 32 of the endoscope 30 is 3.2 mm and the diameter of the body of the forceps 20 is 2.2 mm, resulting in a gap of 1 mm in the diameter direction and 0.5 mm in real terms. In the endoscopic treatment portion traction member 1 according to the present embodiment, the difference between the inner circumferential portion 11 and the peripheral portion 12 of the annular body 10 (i.e., the width or thickness of the annular body 10) is approximately 0.3 to 0.5 mm.

FIG. 4(B) is a cross-sectional view of the endoscope illustrating the portion of the endoscope that includes the forceps 20 and the endoscopic treatment portion traction member 1 gripped by the forceps 20. The annular body 10 of the endoscopic treatment portion traction member 1 in which the outer tongue 15 is gripped by the forceps jaws 21, 22 of the forceps 20 extends in the conduit 32 of the endoscope 30 in accordance with the direction of travel of the forceps 20. As mentioned above, the width (thickness) of the annular body 10 of the endoscopic treatment portion traction member 1 according to the present embodiment is narrower than the gap between the conduit 32 of the endoscope 30 and the forceps 20, allowing the endoscopic treatment portion traction member 1 to pass inside the conduit 32.

As understood from the previous description, the endoscopic treatment portion traction member 1 according to the present embodiment is required to have tensile strength as well as elasticity and flexibility since it is used for traction applications. The elasticity and flexibility are properties required for the forceps 20 to deform in shape and be subject to less resistance when the forceps 20 are moved in and out of the conduit 32 of the endoscope 30. In addition, as shown in FIGS. 5 and 6 below, the endoscopic treatment portion traction member 1 is suspended by clips 60. The endoscopic treatment portion traction member 1 then pulls the clips together. In this case, the material cannot serve as a traction member since it is easily torn when it is pulled by the suspension.

Accordingly, the endoscopic treatment portion traction member 1 is formed by molding an elastic resin. Specifically, the resin may include polypropylene with elastomer components, polyvinyl chloride, urethane resin, and even silicone resin. Especially, silicone resin has excellent corrosion resistance and high stability as a medical material, and thus is desirable in consideration of its applications for treatment in the body. Injection molding, cast molding, or other appropriate methods are used to mold the silicone resin to achieve the endoscopic treatment portion traction member 1.

FIGS. 5 and 6 are schematic diagrams illustrating the form in which the endoscopic treatment portion traction member 1 according to the present embodiment is used in endoscopic submucosal dissection. FIGS. 5 and 6 illustrate a colon 50 as an organ, and the endoscope 30 is inserted through the anus (not shown). FIGS. 5 and 6 illustrate the form in which the lesion site 55 (tumor) in the mucosal tissue 51 on the surface of the colon 50 is resected from the surrounding mucosal tissue 51 as the tissue to be resected.

In FIG. 5(A), a prior visual inspection of the mucosal tissue 51 on the surface of the colon 50 and the inside 52 of the intestinal tract by the endoscope 30 checks the operating field in which the lesion site 55 (tissue to be resected) is present. Saline 53 is injected directly under the lesion site 55 (below the mucosal tissue 51), and the lesion site 55 is lifted from the surrounding mucosal tissue 51. At this time, the clip 60 is inserted into the lesion site 55 through the endoscope 30. The other clip 60 is also inserted into the mucosal tissue 51 on the surface of the colon 50 opposite the clip 60 on the side of the lesion site 55.

As shown in FIG. 5(A), the endoscopic treatment portion traction member 1 is then suspended between the two clips 60, 60, which are inserted into the inside 52 of the intestinal tract. For the suspension of the endoscopic treatment portion traction member 1 between the clips 60, the annular body 10 of the endoscopic treatment portion traction member 1 is inserted into a cut (not shown) in each clip 60 and pushed into the clip 60. This operation is performed through the forceps 20 (forceps jaws 21, 22) inserted into the endoscope 30. The lesion site 55 (tissue to be resected) lifted from the mucosal tissue 51 by the injection of the saline 53 is moderately lifted and maintained by the endoscopic treatment portion traction member 1 connecting the clips 60, 60.

In FIG. 5(B), the mucosal tissue 51 around the lesion site 55 is gradually resected by the electrocautery 35 protruding from the endoscope 30. At this time, the lesion site 55 is lifted by the endoscopic treatment portion traction member 1, facilitating the electrocautery 35 to be applied to the target mucosal tissue 51.

FIG. 6 illustrates the final stage of further performed resection of the lesion site 55 (tissue to be resected). In FIG. 6, the suspension of the endoscopic treatment portion traction member 1 has been adjusted, and the lesion site 55 continues to be resected by the electrocautery 35. As understood from the disclosure and illustration of FIGS. 5 and 6, the endoscopic treatment portion traction member 1 is a traction aid for assisting the traction through the clips.

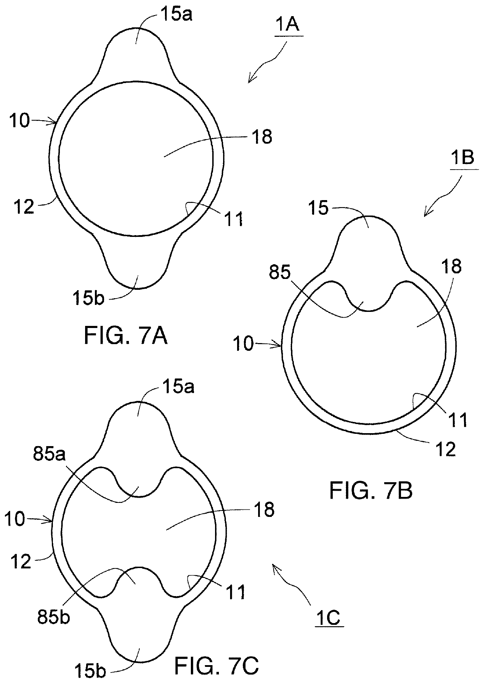

In addition to the endoscopic treatment portion traction member according to the first embodiment that has been illustrated and described so far, the endoscopic treatment portion traction members according to the second through fourteenth embodiments will also be described with reference to FIGS. 7, 8, and 9.

The endoscopic treatment portion traction member 1A according to the second embodiment in FIG. 7(A) includes two outer tongues 15a and 15b at 180° opposite positions of the peripheral portion 12 of the circular annular body 10. In the endoscopic treatment portion traction member 1A, both the outer tongues 15a and 15b are more easily gripped by the forceps.

The endoscopic treatment portion traction member 1B according to the third embodiment in FIG. 7(B) includes an outer tongue 15 in the peripheral portion 12 of the circular annular body 10 and an inner tongue 85 in the inner circumferential portion 11 of the annular body 10 opposite the outer tongue 15. The inner tongue 85 is provided to be more easily grasped by the forceps.

The endoscopic treatment portion traction member 1C according to the fourth embodiment in FIG. 7(C) includes two outer tongues 15a and 15b at 180° opposite positions of the peripheral portion 12 of the circular annular body 10. Inner tongues 85a and 85b are further provided in the inner circumferential portion 11 at opposite positions of both the outer tongues 15a and 15b. The tongues at both ends allow the endoscopic treatment portion traction member to be easily gripped.

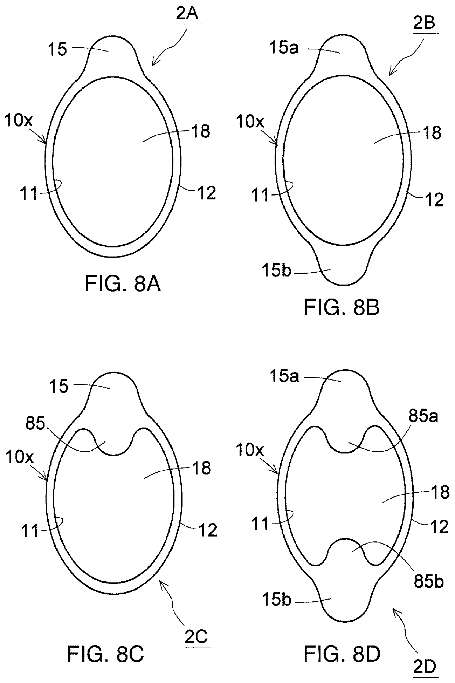

Each endoscopic treatment portion traction member in FIG. 8 includes an oval-shaped annular body 10x. The ratio of the minor axis to the major axis of the oval-shaped annular body 10x is appropriate. In consideration of practicality, the ratio of the minor axis to the major axis (minor axis:major axis) is 1:1.1 to 1:4. The maximum length of the outer tongue 15 (15a, 15b) is in the range of 1/10 to 3/10 of the minor axis length of the peripheral portion 12 of the oval-shaped annular body 10x. The same may be applied to each of the forms in FIG. 8.

The endoscopic treatment portion traction member 2A according to the fifth embodiment in FIG. 8(A) includes an oval-shaped annular body 10x, and an outer tongue 15 is provided at one location in the peripheral portion 12 of the annular body 10x. The oval shape of the annular body allows the direction of the endoscopic treatment portion traction member to be visually easily determined when it is gripped by the forceps or inserted into the endoscope.

The endoscopic treatment portion traction member 2B according to the sixth embodiment in FIG. 8(B) includes two outer tongues 15 at 180° opposite positions of the peripheral portion 12 of the oval-shaped annular body 10x. In the endoscopic treatment portion traction member 2B, both the outer tongues 15a and 15b are more easily gripped by the forceps.

The endoscopic treatment portion traction member 2C according to the seventh embodiment in FIG. 8(C) includes an outer tongue 15 in the peripheral portion 12 of the oval-shaped annular body 10 and an inner tongue 85 in the inner circumferential portion 11 of the annular body 10x opposite the outer tongue 15. The inner tongue 85 is provided to be more easily grasped by the forceps.

The endoscopic treatment portion traction member 2D according to the eighth embodiment in FIG. 8(D) includes two outer tongues 15a and 15b at 180° opposite positions of the peripheral portion 12 of the oval-shaped annular body 10x. Inner tongues 85a and 85b are further provided in the inner circumferential portion 11 at opposite positions of both the outer tongues 15a and 15b. The tongues at both ends allow the endoscopic treatment portion traction member to be easily gripped.

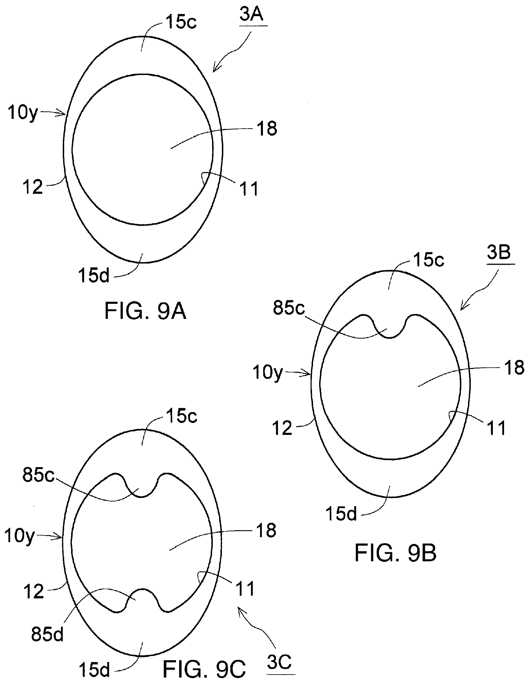

Each endoscopic treatment portion traction member in FIG. 9 is an oval-shaped annular body 10y, and a variation of FIG. 8. That is, the outer tongue 15 (15c, 15d) is not protruding but is incorporated into a part of the oval-shaped annular body 10y. In FIG. 9, the ratio of the minor axis to the major axis of the oval-shaped annular body 10y is also appropriate. In consideration of practicality, the ratio of the minor axis to the major axis (minor axis:major axis) is 1:1.1 to 1:4. The maximum length of the outer tongue 15 (15c, 15d) is in the range of 1/10 to 3/10 of the minor axis length of the peripheral portion 12 of the oval-shaped annular body 10y. The same may be applied to each of the forms in FIG. 9.

In the endoscopic treatment portion traction member 3A according to the ninth embodiment in FIG. 9(A), the width of the outer tongues 15c and 15d is extended and integrated with the oval-shaped annular body 10y. The same may be applied to the other forms in FIG. 9. The endoscopic treatment portion traction member 3A has less resistance in terms of shape, facilitating the insertion into the endoscope.

The endoscopic treatment portion traction member 3B according to the tenth embodiment in FIG. 9(B) includes outer tongues 15c and 15d with the widths extended at opposite positions in the peripheral portion 12 of the oval-shaped annular body 10y and an inner tongue 85c in the inner circumferential portion 11 of the annular body 10y opposite the outer tongue 15c. The inner tongue 85 is provided to be more easily grasped by the forceps.

The endoscopic treatment portion traction member 3C according to the eleventh embodiment in FIG. 9(C) includes outer tongues 15c and 15d with the widths extended at opposite positions in the peripheral portion 12 of the oval-shaped annular body 10y and inner tongues 85c and 85d in the inner circumferential portion 11 of the annular body 10y opposite the outer tongues 15c and 15d. The inner tongues 85c and 85d facilitate gripping by the forceps from both directions.

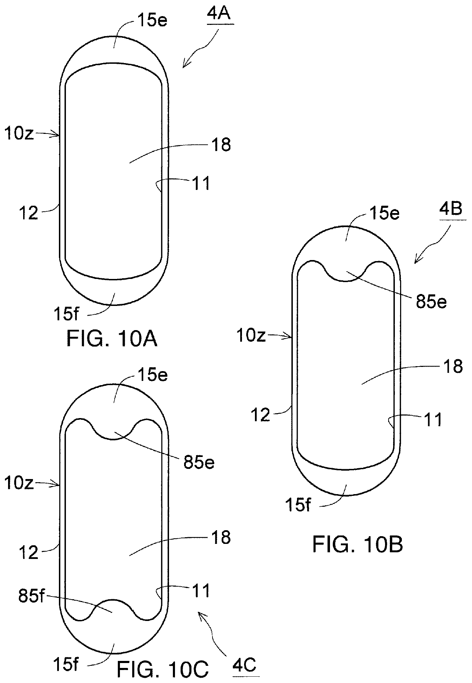

Each endoscopic treatment portion traction member in FIG. 10 is a deformed oval-shaped annular body 10z, and includes a shape referred to as a stadium track, for example. In FIG. 10, the outer tongues 15e, 15f are not protruding but are incorporated into a part of the oval-shaped annular body 10z. In FIG. 10, the ratio of the minor axis to the major axis of the annular body 10z is also appropriate. In consideration of practicality, the ratio of the minor axis to the major axis (minor axis:major axis) is 1:1.1 to 1:4. The maximum length of the outer tongue 15 (15e, 15f) is in the range of 1/10 to 3/10 of the minor axis length of the peripheral portion 12 of the oval-shaped annular body 10z. The same may be applied to each of the forms in FIG. 10.

In the endoscopic treatment portion traction member 4A according to the twelfth embodiment in FIG. 10(A), the width of the outer tongues 15e and 15f is extended and integrated with the oval-shaped annular body 10z. The same may be applied to the other forms in FIG. 10. The endoscopic treatment portion traction member 4Ahas less resistance in terms of shape, facilitating the insertion into the endoscope.

The endoscopic treatment portion traction member 4B according to the thirteenth embodiment in FIG. 10(B) includes outer tongues 15e and 15f with the widths extended at opposite positions in the peripheral portion 12 of the oval-shaped annular body 10z and an inner tongue 85e in the inner circumferential portion 11 of the annular body 10z opposite the outer tongue 15e. The inner tongue 85e is provided to be more easily grasped by the forceps.

The endoscopic treatment portion traction member 4C according to the fourteenth embodiment in FIG. 10(D) includes outer tongues 15e and 15f with the widths extended at opposite positions in the peripheral portion 12 of the oval-shaped annular body 10z and inner tongues 85e and 85f in the inner circumferential portion 11 of the annular body 10z opposite the outer tongues 15e and 15f. The inner tongues 85e and 85f facilitate gripping by the forceps from both directions.

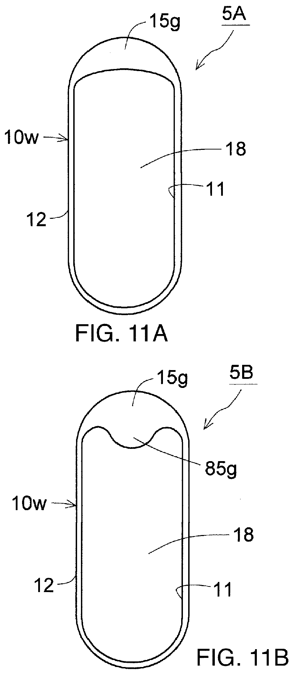

Each endoscopic treatment portion traction member in FIG. 11 is a deformed oval-shaped annular body 10w, and corresponds to a variation of the annular body 10z (with the shape referred to as a stadium track, for example) in FIG. 10. The outer tongue 15g is located at only one position, and is not protruding, but rather is incorporated into a portion of the oval-shaped annular body 10w. In FIG. 11, the ratio of the minor axis to the major axis of the annular body 10w is also appropriate. In consideration of practicality, the ratio of the minor axis to the major axis (minor axis:major axis) is 1:1.1 to 1:4. The maximum length of the outer tongue 15 (15g) is in the range of 1/10 to 3/10 of the minor axis length of the peripheral portion 12 of the oval-shaped annular body 10w. The same may be applied to each of the forms in FIG. 11.

In the endoscopic treatment portion traction member 5A according to the fifteenth embodiment in FIG. 11(A), the width of the outer tongue 15g is extended and integrated with the oval-shaped annular body 10w. The same may be applied to the other forms in FIG. 11. The endoscopic treatment portion traction member 5A has less resistance in terms of shape, facilitating the insertion into the endoscope. The arrangement of the outer tongue 15g also determines the directionality of the endoscopic treatment portion traction member 5A at a time when it is gripped by the forceps.

In the endoscopic treatment portion traction member 5B according to the sixteenth embodiment in FIG. 11(B), the width of the outer tongue 15g is extended and integrated with the oval-shaped annular body 10w. Further, an inner tongue 85g is provided in the inner circumferential portion 11 of the annular body 10w opposite the outer tongue 15g. The inner tongue 85g is provided to be more easily grasped by the forceps. In the same manner, the arrangement of the outer tongue 15g determines the directionality of the endoscopic treatment portion traction member 5A at a time when it is gripped by the forceps.

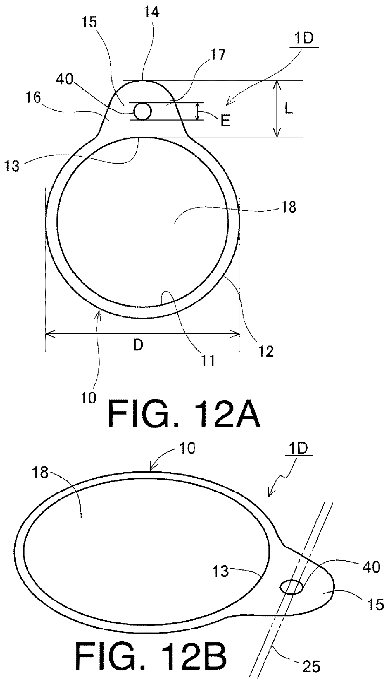

For each of the endoscopic treatment portion traction members illustrated in detail in FIGS. 1 through 11, a tongue pore may also be formed into the outer tongue. FIG. 12(A) is a plan view of an endoscopic treatment portion traction member 1D according to the seventeenth embodiment. The outer tongue 15 of the endoscopic treatment portion traction member 1D is perforated (formed therethrough) with a tongue pore 40. As shown in a perspective view in FIG. 12(B), the forceps tip end 25 of forceps with a form different from that of the forceps 20 (see FIGS. 2 to 4) is inserted into the tongue pore 40. The forceps tip end 25 shown in the FIG. 12(B) corresponds to a thin rod-shaped member and is illustrated in part. Two forceps tip ends 25 can be crossed (not shown). As described above, the endoscopic treatment portion traction member 1D is hooked to the forceps tip end 25 through the tongue pore 40. In addition, if needed, the forceps tip end 25 side may include a plastic annular spacer member (not shown) to protect the contact area with the tongue pore 40. This is to protect the metal forceps tip end 25 and the plastic endoscopic treatment portion traction member 1D from damage due to wear.

The tongue pore 40 may have an opening large enough to ensure the insertion of the forceps tip end 25. The maximum length (E) of the tongue pore 40 is specified in the range of 0.5/10 to 2/10, preferably 1/10 to 2/10 of the diameter of the peripheral portion 12 of the annular body 10. Insertion of the forceps tip end 25 into the tongue pore 40 is less smooth. In addition, if the size is considerably large, the connection portion between the outer tongue 15 and the annular body 10 is likely to decrease, resulting in a reduction in strength. For example, in the case of the endoscopic treatment portion traction member 1D according to the seventeenth embodiment, the diameter (D) of the peripheral portion 12 of the annular body 10 is 10 mm and the maximum length (E) of the tongue pore 40 is 1.5 mm (1.5/10 of the diameter of the peripheral portion of the annular body).

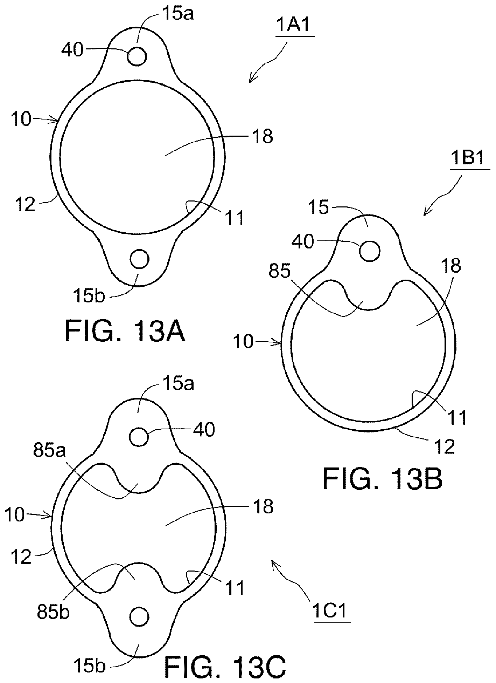

The formation of the tongue pore in the outer tongue may be applied to any of the endoscopic treatment portion traction members according to the second to sixteenth embodiments disclosed in FIGS. 7 to 11. Therefore, each endoscopic treatment portion traction member with a tongue pore in the outer tongue is shown as in FIGS. 13 to 17. Since the configuration other than the tongue pore 40 is not changed in each endoscopic treatment portion traction member, the description thereof is omitted mainly as an illustration.

FIG. 13(A) illustrates an endoscopic treatment portion traction member 1A1 according to the eighteenth embodiment, FIG. 13(B) illustrates an endoscopic treatment portion traction member 1B1 according to the nineteenth embodiment, and FIG. 13(C) illustrates an endoscopic treatment portion traction member 1C1 according to the twentieth embodiment. The endoscopic treatment portion traction members 1A1, 1B1, and 1C1 are capable of accommodating both the gripping of the outer tongue 15 by the forceps 20 or the insertion of the forceps tip end 25 into the tongue pore 40.

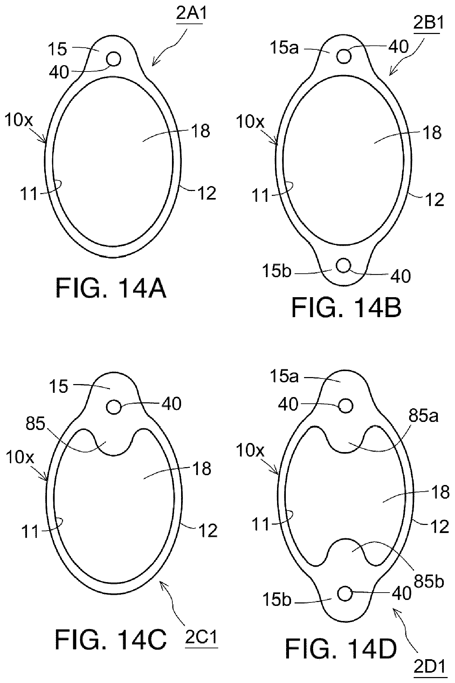

FIG. 14(A) illustrates an endoscopic treatment portion traction member 2A1 according to the twenty-first embodiment, FIG. 14(B) illustrates an endoscopic treatment portion traction member 2B1 according to the twenty-second embodiment, FIG. 14(C) illustrates an endoscopic treatment portion traction member 2C1 according to the twenty-third embodiment, and FIG. 14(D) illustrates an endoscopic treatment portion traction member 2D1 according to the twenty-fourth embodiment. Even the endoscopic treatment portion traction members 2A1, 2B1, 2C1, and 2D1 with oval-shaped annular bodies are capable of accommodating both the gripping of the outer tongue 15 by the forceps 20 or the insertion of the forceps tip end 25 into the tongue pore 40.

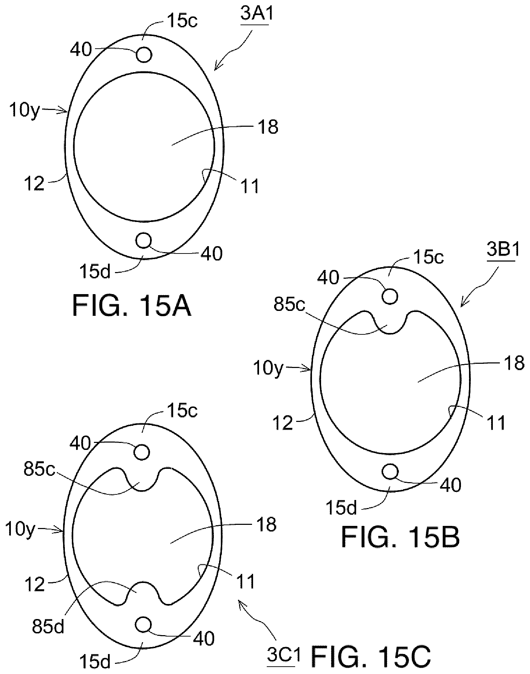

FIG. 15(A) illustrates an endoscopic treatment portion traction member 3A1 according to the twenty-fifth embodiment, FIG. 15(B) illustrates an endoscopic treatment portion traction member 3B1 according to the twenty-sixth embodiment, and FIG. 15(C) illustrates an endoscopic treatment portion traction member 3C1 according to the twenty-seventh embodiment. Even the endoscopic treatment portion traction members 3A1, 3B1, and 3C1 with oval-shaped annular bodies in which the widths of the outer tongues are extended are capable of accommodating both the gripping of the outer tongue 15 by the forceps 20 or the insertion of the forceps tip end 25 into the tongue pore 40.

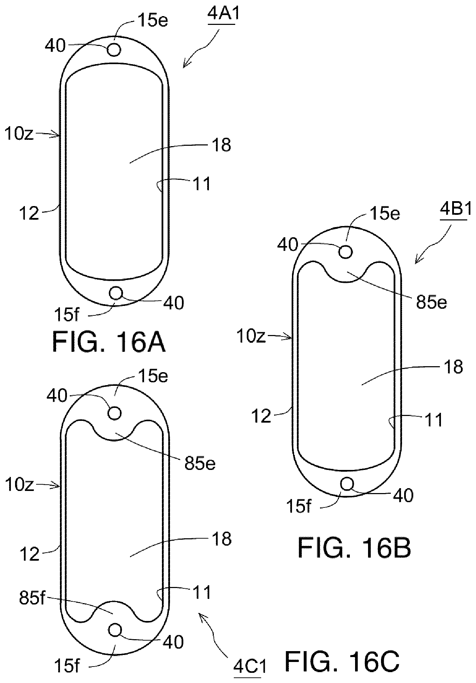

FIG. 16(A) illustrates an endoscopic treatment portion traction member 4A1 according to the twenty-eighth embodiment, FIG. 16(B) illustrates an endoscopic treatment portion traction member 4B1 according to the twenty-ninth embodiment, and FIG. 16(C) illustrates an endoscopic treatment portion traction member 4C1 according to the thirtieth embodiment. The modified oval-shaped endoscopic treatment portion traction members 4A1, 4B1, and 4C1 are capable of accommodating both the gripping of the outer tongue 15 by the forceps 20 or the insertion of the forceps tip end 25 into the tongue pore 40.

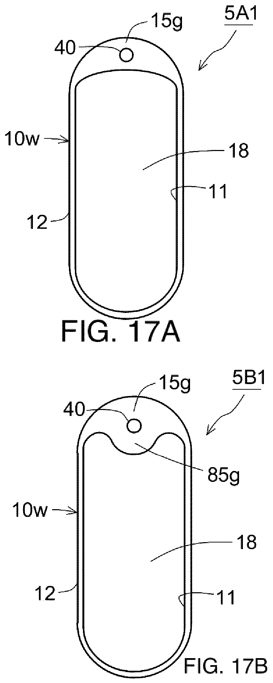

FIG. 17(A) illustrates an endoscopic treatment portion traction member SA1 according to the thirty-first embodiment, and FIG. 17(B) illustrates an endoscopic treatment portion traction member 5B1 according to the thirty-second embodiment. The oval-shaped endoscopic treatment portion traction members SA1 and 5B1, which are deformed in the same manner as in FIG. 16, are capable of accommodating both the gripping of the outer tongue 15 by the forceps 20 or the insertion of the forceps tip end 25 into the tongue pore 40.

### [Industrial Applicability]

The endoscopic treatment portion traction member has improved ease of use with endoscopic forceps in comparison to the existing traction members. Therefore, it is promising as a traction member for procedures such as endoscopic submucosal dissection.

### [Description of the Reference Numerals]

1, 1A, 1B, 1C, 2A, 2B, 2C, 2D, 3A, 3B, 3C, 4A, 4B, 4C, 5A, 5B, 1D, 2A1, 2B1, 2C1, 2D1, 3A1, 3B1, 3C1, 4A1, 4B1, 4C1, 5A1, 5B1: Endoscopic Treatment Portion Traction Member
10, 10x, 10y, 10z, 10w: Annular Body
11: Inner Circumferential Portion
12: Peripheral Portion
13: Installation Position
14: Tip End
15, 15a, 15b, 15c, 15d, 15e, 15f, 15g: Outer Tongue
16, 17: Tapered Portion
18: Inner Circular Portion
20: Forceps
21, 22: Forceps Jaws
25: Forceps Tip End
30: Endoscope
31: Insertion Port
32: Conduit
33: Opening
35: Electrocautery
36: Loop End
40: Tongue Pore
50: Colon
51: Mucosal Tissue
52: Inside of Intestinal Tract
55: Lesion Site (Tissue to be Resected)
60: Clips
85, 85a, 85b, 85c, 85d, 85e, 85f, 85g: Inner Tongue
D: Diameter of Peripheral Portion of Annular Body
L: Maximum Length of Tongue
E: Maximum Length of Tongue Pore

## Claims

1. An endoscopic treatment portion traction member that is gripped by forceps to be inserted into an endoscope and then inserted into a body, and is fixed to a tissue to be resected and pulls the tissue from a surrounding tissue, the endoscopic treatment portion traction member comprising:
an annular body; and,
an outer tongue provided in a peripheral portion of the annular body and is gripped by forceps.

2. The endoscopic treatment portion traction member according to claim 1, wherein the outer tongue is provided at one or two locations in the peripheral portion of the annular body.

3. The endoscopic treatment portion traction member according to claim 1 or 2, wherein the outer tongue has a tapered portion.

4. The endoscopic treatment portion traction member according to any one of claims 1 to 3, wherein a tip end of the outer tongue is chamfered in a shape of a circular arc.

5. The endoscopic treatment portion traction member according to any one of claims 1 to 4, wherein the annular body is circular in shape and a maximum length of the outer tongue is 1/10 to 6/10 of a diameter of the peripheral portion of the circular annular body.

6. The endoscopic treatment portion traction member according to any one of claims 1 to 4, wherein the annular body is oval in shape and a maximum length of the outer tongue is 1/10 to 3/10 of a minor axis length of the peripheral portion of the oval-shaped annular body.

7. The endoscopic treatment portion traction member according to any one of claims 1 to 5, wherein the annular body is circular in shape and a diameter of the peripheral portion of the annular body is 5 to 30 mm.

8. The endoscopic treatment portion traction member according to any one of claims 1 to 7, wherein an inner tongue is provided on an inner circumferential portion of the annular body opposite the outer tongue.

9. The endoscopic treatment portion traction member according to any one of claims 1 to 8, wherein the endoscopic treatment portion traction member is formed from elastic resin.

10. The endoscopic treatment portion traction member according to any one of claims 1 to 9, wherein the outer tongue is formed with a tongue pore.

11. The endoscopic treatment portion traction member according to any one of claims 1 to 10, wherein when the endoscopic treatment portion traction member is fixed to the tissue to be resected, the endoscopic treatment portion traction member is suspended via a clip secured to the tissue to be resected.
